# EUROPEAN PATENT APPLICATION

(11) **EP 4 613 732 A1**
(43) Date of publication of application: **10.09.2025**
(21) Application number: 23885620.7
(22) Date of filing: 25.10.2023
(51) Int. Cl.: C07C 57/15, C07C 63/24, C07C 63/26, C07C 63/307, C07C 63/331, C07C 229/62, C07C 323/62, C07D 207/34, C07D 213/22, C07D 307/68, C07F 15/06, C07F 3/06, C07F 5/00, C07F 5/06, C07F 7/00, C07F 7/28

(54) **METAL-ORGANIC FRAMEWORK**

(30) Priority: 31.10.2022 JP 2022174075; 31.10.2022 JP 2022174076
(71) Applicant: Kyoto University, Kyoto-shi, Kyoto 606-8501 (JP); Sumitomo Chemical Company, Limited, Tokyo 103-6020 (JP)
(72) Inventor: KITAGAWA, Susumu, Kyoto-shi, Kyoto 606-8501 (JP); HIGUCHI, Masakazu, Kyoto-shi, Kyoto 606-8501 (JP); OTAKE, Kenichi, Kyoto-shi, Kyoto 606-8501 (JP); KIKUCHI, Yuta, Ichihara-shi, Chiba 299-0195 (JP)
(74) Representative: Vossius & Partner Patentanwälte Rechtsanwälte mbB
(86) International application number: PCT/JP2023/038535
(87) International publication number: WO 2024/095863

(57) **Abstract**

An object of the present invention is to provide a MOF which is highly water-adsorbent even in an environment with low relative pressure. The present invention is a metal organic framework comprising a predetermined organic ligand and metal ion, wherein a ratio of a largest pore diameter L to a smallest pore diameter S (the largest pore diameter L/the smallest pore diameter S) determined through the following procedures (a1) to (a3) is 1.0 to 2.0, and a decomposition start temperature is higher than 200°C.
(a1) a crystal structure determined through X-ray crystal structure analysis is displayed using a space-filling model, and presence or absence of the through pores is observed under a predetermined condition, to identify a through pore Pₘₐₓ having the largest inscribed circle diameter;
(a2) the longest length is found among inner diameters of the through pore Pₘₐₓ in the predetermined cross section, to specify the largest pore diameter L of the through pore Pₘₐₓ,
(a3) in the cross section in which the largest pore diameter L is determined, the smallest pore diameter S is specified.

## Description

### TECHNICAL FIELD

The present invention relates to a metal organic framework.

### BACKGROUND ART

Metal organic frameworks are also referred to as porous coordination polymers and are a class of materials which form porous structures by coordinate bonds between metal ions and organic ligands, and are expected to adsorb and desorb gas and expected to be applied to catalysts, etc.

For example, Patent Literature 1 discloses a metal organic framework including metal ions, first ligands, second ligands, and optional third ligands. In the metal organic framework: the metal ion is an aluminum ion; the first and second ligands are each an ion of an organic compound that includes a hetero ring having two carboxyl groups; a heteroatom and the angle formed between the carboxyl groups satisfy a predetermined condition; the third ligand is an ion of an organic compound having two carboxyl groups; and the proportion of each of the existing first to third ligands is within a predetermined range.

### CITATION LIST

### PATENT LITERATURE

PTL 1: Japanese Laid-Open Patent Publication No. 2020-176101

### SUMMARY OF THE INVENTION

### PROBLEMS TO BE SOLVED BY THE INVENTION

The metal organic framework (hereinafter, may be referred to as MOF) is required to be highly adsorbent even in an environment in which relative pressure: P/P₀ (P represents water vapor pressure and P₀ represents saturation water vapor pressure) is low, depending on the application.

Therefore, an object of the present invention is to provide a MOF which is highly water-adsorbent even in an environment with low relative pressure.

### SOLUTION TO THE PROBLEMS

The present invention that can achieve the problem is as follows.
[1] A metal organic framework comprising
   an organic ligand and a metal ion, wherein
   the organic ligand includes an organic ligand having a group represented by R(COO⁻)ₙ in which R represents at least one of an unsaturated straight-chain hydrocarbon group, an aromatic hydrocarbon group, and a hydrocarbon group that has an aromatic ring including an oxygen atom and may have a functional group X which is -OH or -S-S-, and n is 2 or more and 3 or less,
   the metal ion is an ion of at least one metal selected from the group consisting of Al, Ga, In, Ti, V, Cr, Mn, Fe, Co, Cu, Zr, and Hf,
   a ratio of a largest pore diameter L to a smallest pore diameter S (the largest pore diameter L/the smallest pore diameter S) determined through the following procedures (a1) to (a3) is 1.0 to 2.0, and
   a decomposition start temperature is higher than 200°C,
   (a1) a crystal structure determined through X-ray crystal structure analysis is displayed using a space-filling model, and is displayed so as to include outer perimeters of through pores without any omissions, and presence or absence of the through pores is observed from all directions, to identify a through pore Pₘₐₓ having the largest inscribed circle diameter;
   (a2) in each of a cross section ab, a cross section bc, and a cross section ca of the through pore Pₘₐₓ, a longest length is found among inner diameters of the through pore Pₘₐₓ obtained by cutting the through pore Pₘₐₓ along lines orthogonal to a central axis of the through pore Pₘₐₓ, and a longest one, among the longest length in the cross section ab, the longest length in the cross section bc, and the longest length in the cross section ca, is set as the largest pore diameter L of the through pore Pₘₐₓ,
   the cross section ab being a cut cross section, of the through pore Pₘₐₓ, along a plane parallel to an a-b plane of a unit cell, and being a plane obtained by cutting the through pore Pₘₐₓ where the through pore Pₘₐₓ penetrates,
   if a plurality of the cross sections along which the through pore Pₘₐₓ can be cut where the through pore Pₘₐₓ penetrates are present, the cross section ab being the cross section in which an overlapping portion with the through pore Pₘₐₓ is the largest,
   the cross section bc being a cross section obtained by replacing the cross section ab with the cross section bc and the a-b plane with a b-c plane, and the cross section ca being a cross section obtained by replacing the cross section ab with the cross section ca and the a-b plane with a c-a plane, in identifying of the cross section ab; and
   (a3) in the cross section in which the largest pore diameter L is determined, a smallest length, among inner diameters of the through pore Pₘₐₓ obtained by cutting the through pore Pₘₐₓ along the lines orthogonal to the central axis of the through pore Pₘₐₓ, is set as the smallest pore diameter S.
[2] The metal organic framework according to [1], wherein
   a through pore observed when a crystal structure of a metal organic framework determined through X-ray crystal structure analysis is displayed using a space-filling model does not intersect with another through pore.

### ADVANTAGEOUS EFFECTS OF THE INVENTION

According to the present invention, the MOF capable of adsorbing a sufficient amount of water even in an environment with low relative pressure can be provided.

### BRIEF DESCRIPTION OF THE DRAWINGS

[FIG. 1] FIG. 1 shows an example in which a crystal structure of a MOF is displayed using a space-filling model and is observed from one direction.
[FIG. 2] FIG. 2 shows an example in which the crystal structure of the MOF is displayed using the space-filling model and is observed from another direction.
[FIG. 3] FIG. 3 shows an example in which the crystal structure of the MOF is displayed using the space-filling model and is observed from still another direction.
[FIG. 4] FIG. 4 shows a largest pore diameter L and a smallest pore diameter S of a through pore Pₘₐₓ.

### DESCRIPTION OF EMBODIMENTS

A MOF of the present invention includes an organic ligand and a metal ion. The ratio of a largest pore diameter L to a smallest pore diameter S (the largest pore diameter L/the smallest pore diameter S) determined through predetermined procedures is 1.0 to 2.0, and a decomposition start temperature is higher than 200°C.

### 1. Ratio of largest pore diameter L to smallest pore diameter S (largest pore diameter L/smallest pore diameter S)

The MOF of the present invention has the largest pore diameter L/the smallest pore diameter S of 1.0 to 2.0. When the largest pore diameter L/the smallest pore diameter S is in the above range, each shape of pores has few twists and turns, so that water can be favorably adsorbed even if relative pressure is low. The largest pore diameter L/the smallest pore diameter S is preferably 1.03 or more, more preferably 1.05 or more, and further preferably 1.10 or more, and is preferably 1.6 or less, more preferably 1.5 or less, and further preferably 1.4 or less. Furthermore, the largest pore diameter L/the smallest pore diameter S may be 1.20 or more and 1.50 or less.

The largest pore diameter L/the smallest pore diameter S is determined through the following procedures (a1) to (a3).

(a1) A crystal structure determined through X-ray crystal structure analysis is displayed using a space-filling model, and is displayed so as to include the outer perimeters of through pores without any omissions, and the presence or absence of the through pores is observed from all directions, to identify a through pore Pₘₐₓ having the largest inscribed circle diameter.

As in the above-described measurement of the porosity, the X-ray crystal structure analysis is performed using XRD measurement to obtain a cif, and the cif is loaded into the Mercury, whereby a single space-filling model (crystal structure) is obtained.

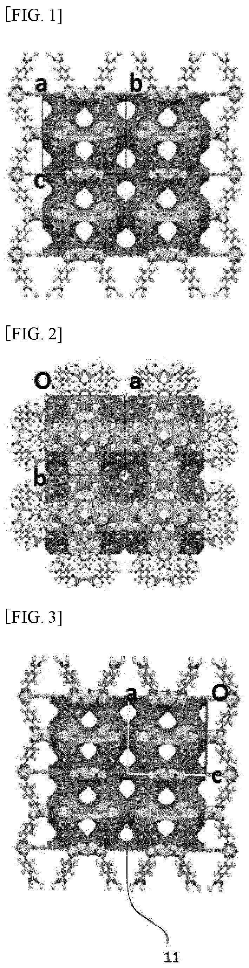

After the space-filling model of the crystal structure has been obtained, the crystal structure is displayed so as to include the outer perimeters of the through pores without any omissions and is rotated, and then the presence or absence of the through pores is observed from all directions. FIG. 1 to FIG. 3 each show through pores observed from a predetermined direction by rotating the obtained crystal structure. FIG. 3 shows an inscribed circle 11 of the through pore observed from a direction shown in FIG. 3. The inscribed circles of all the through pores observed from each direction are identified, and a through pore Pₘₐₓ having the largest inscribed circle diameter is identified among all the through pores observed.

In the procedure (a1), in order to observe all the through pores so as to include the outer perimeters without any omissions, observation of a unit cell is performed first. If the through pore with a missing outer perimeter is present, a 2×2×2 expansion of the unit cell may be displayed and observed.

(a2) Next, the through pore Pₘₐₓ is observed in each of a specific cross section ab, a specific cross section bc, and a specific cross section ca as described below.

The cross section ab is a cut cross section, of the through pore Pₘₐₓ, along a plane parallel to an a-b plane of a unit cell, and is a plane obtained by cutting the through pore Pₘₐₓ where the through pore Pₘₐₓ penetrates. In some cases, a plurality of cross sections that are parallel to the a-b plane of the unit cell and along which the through pore Pₘₐₓ can be cut where the through pore Pₘₐₓ penetrates, are present. If the plurality of cross sections are present, the cross section ab is a cross section in which the overlapping portion with the through pore Pₘₐₓ is the largest.

The cross section bc is a cross section obtained by replacing the cross section ab with the cross section bc and the a-b plane with a b-c plane, in identifying of the cross section ab.

The cross section ca is a cross section obtained by replacing the cross section ab with the cross section ca and the a-b plane with a c-a plane.

An observation region in each of these cross sections ab, bc, ca corresponds to that in the procedure (a1).

After the through pore Pₘₐₓ is observed in each of the cross section ab, the cross section bc, and the cross section ca, the longest length is found among inner diameters of the through pore Pₘₐₓ obtained by cutting the through pore Pₘₐₓ along lines orthogonal to the central axis of the through pore Pₘₐₓ, and the longest one, among the longest length in the cross section ab, the longest length in the cross section bc, and the longest length in the cross section ca, is set as the largest pore diameter L of the through pore Pₘₐₓ.

(a3) Further, in the cross section in which the largest pore diameter L is determined, the smallest length, among inner diameters of the through pore Pₘₐₓ obtained by cutting the through pore Pₘₐₓ along the lines orthogonal to the central axis of the through pore Pₘₐₓ, is set as the smallest pore diameter S.

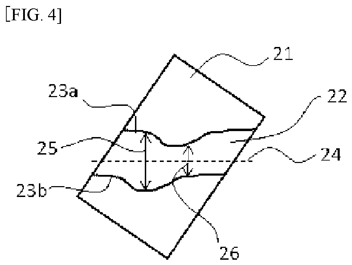

FIG. 4 shows a cross section 21 (one of the cross section ab, the cross section bc, and the cross section ca) in which a largest pore diameter L25 of a through pore Pₘₐₓ 22 is determined. Reference characters 23a, 23b represent the outer perimeter of the through pore Pₘₐₓ 22, and a reference character 24 represents the central axis of the through pore Pₘₐₓ 22. In the cross section 21 in which the largest pore diameter L25 is determined, a reference character 26 represents the smallest length among inner diameters of the through pore Pₘₐₓ 22 obtained by cutting the through pore Pₘₐₓ 22 along lines orthogonal to the central axis 24 of the through pore Pₘₐₓ 22, and is the smallest pore diameter S.

The value of the largest pore diameter L and the value of the smallest pore diameter S are not limited as long as the largest pore diameter L/the smallest pore diameter S is 1.0 to 2.0, and the largest pore diameter L is 2.5 to 20 Å and the smallest pore diameter S is 1.5 to 15 Å, for example.

From the viewpoint of water adsorption amount, it is preferable that a through pore observed using the space-filling model does not intersect with another through pore.

### 2. Decomposition start temperature

As in Examples described below, the MOF was held at 25°C for 12 hours under an air atmosphere in which the relative humidity was adjusted to 50% to perform pre-treatment. Then, the temperature was increased to 500°C at a temperature increase rate of 5°C/min. while nitrogen was caused to flow. According to DTA measurement defined in General rules for thermal analysis of JIS K0129, a point at which decomposition behavior was observed in this section (25 to 500°C) was set as a decomposition start temperature. That the decomposition start temperature is high means that the bond between the metal ion and the organic ligand is strong and that the distance between the metal ion and the organic ligand is short. It is inferred that, when this distance is short, electrons on the organic ligand side are attracted toward the metal side in the bond between the metal ion and oxygen derived from the organic ligand, so that the state of electron deficiency on the organic ligand side is caused, and this makes it easier for an unshared electron pair on an oxygen atom of a water molecule to bond to the electron-deficient portion, whereby the MOF becomes more water-adsorbent.

The decomposition start temperature is preferably 210°C or higher, more preferably 220°C or higher, and further preferably 230°C or higher, and may be 300°C or lower or 280°C or lower. The decomposition start temperature may be 245°C or higher and 265°C or lower.

The metal ion constituting the MOF is an ion of at least one metal selected from the group consisting of Al, Ga, In, Ti, V, Cr, Mn, Fe, Co, Cu, Zr, and Hf, is more preferably an ion of at least one metal selected from the group consisting of Al, Ga, In, Ti, V, Co, Zr, and Hf, is further preferably an ion of at least one metal selected from the group consisting of Al, Co, and Zr, is even more preferably an Al ion and/or a Zr ion, and is particularly preferably a Zr ion.

The organic ligand constituting the MOF includes at least one selected from the group consisting of carboxylates (carboxylato) represented by R(COO⁻)ₙ (R represents an n-valent group, and n represents an integer of 2 or more and 3 or less). R represents at least one of an unsaturated straight-chain hydrocarbon group, an aromatic hydrocarbon group, and a hydrocarbon group that has an aromatic ring including an oxygen atom, and may have a functional group X which is -OH or -S-S-.

As the unsaturated straight-chain hydrocarbon group, an unsaturated straight-chain hydrocarbon group having 2 to 24 carbon atoms is preferable, an unsaturated straight-chain hydrocarbon group having 2 to 12 carbon atoms is more preferable, an unsaturated straight-chain hydrocarbon group having 2 to 4 carbon atoms is further preferable, and -CH=CH- is particularly preferable.

As the aromatic hydrocarbon group, an aromatic hydrocarbon group that has 6 to 30 carbon atoms and that may include a functional group X which is -OH or -S-S- is preferable, an aromatic hydrocarbon group that has 6 to 14 carbon atoms and that may include a functional group X which is -OH or -S-S- is more preferable, an aromatic hydrocarbon group that has 6 to 12 carbon atoms and that may include a functional group X which is -OH or -S-S- is further preferable, and specific examples thereof include a group that is obtained by removing two or three hydrogen atoms from benzene or biphenyl and that may include a functional group X which is -OH or -S-S-.

Examples of the hydrocarbon group that has an aromatic ring including an oxygen atom and that may have a functional group X which is -OH or -S-S- (particularly, -OH), include furan, oxazole, and tetrahydropyran (i.e., a group obtained by removing two or three hydrogen atoms from furan, oxazole, or tetrahydropyran), each of which may have a functional group X which is -OH, and furan (i.e., a group obtained by removing two or three hydrogen atoms from furan) is particularly preferable.

The R is preferably at least one of: an unsaturated straight-chain hydrocarbon group; an aromatic hydrocarbon group, which may have a functional group X which is -OH or -S-S-; and a hydrocarbon group that has an aromatic ring including an oxygen atom, and is more preferably at least one of: an unsaturated straight-chain hydrocarbon group having 2 to 4 carbon atoms; an aromatic hydrocarbon group that has 6 to 12 carbon atoms and that may have a functional group X which is -OH or -S-S-; and furan, oxazole, or tetrahydropyran, each of which is a hydrocarbon group that has an aromatic ring including an oxygen atom.

The number of carbon atoms in the R is preferably 2 or more, and is preferably 30 or less, more preferably 24 or less, further preferably 18 or less, and still more preferably 12 or less.

The organic ligand constituting the MOF includes at least one selected from the group consisting of carboxylate represented by R(COO⁻)₂ and carboxylate represented by R(COO⁻)₃ (R is equivalent to the R described above), and specific examples thereof include carboxylate in which two protons have been dissociated from two carboxyl groups (-COOH) of dicarboxylic acid, and carboxylate in which three protons have been dissociated from three carboxyl group of tricarboxylic acid.

Examples of the dicarboxylic acid include fumaric acid, 1,4-butenedicarboxylic acid, acetylene dicarboxylic acid, 1,2-benzenedicarboxylic acid (phthalic acid), 1,3-benzenedicarboxylic acid (isophthalic acid), 1,3-butadiene-1,4-dicarboxylic acid, 1,4-benzenedicarboxylic acid (terephthalic acid), 2,5-dihydroxy terephthalic acid, 2,2'-dithiodibenzoic acid, perylene-3,9-dicarboxylic acid, 4,4'-dihydroxybiphenyl-3,3'-dicarboxylic acid, 1,1'-binaphthyl dicarboxylic acid, phenylindandicarboxylic acid, naphthalene-1,8-dicarboxylic acid, 1,4-naphthalene dicarboxylic acid, 2,6-naphthalene dicarboxylic acid, 2,3-naphthalene dicarboxylic acid, anthracene-2,3-dicarboxylic acid, 2',3'-diphenyl-p-terphenyl-4,4"-dicarboxylic acid, 5-tert-butyl-1,3-benzenedicarboxylic acid, 5-hydroxy-1,3-benzenedicarboxylic acid, 2,5-dihydroxy-1,4-benzenedicarboxylic acid, furan-2,5-dicarboxylic acid, 1-nonene-6,9-dicarboxylic acid, and eicosene dicarboxylic acid. The dicarboxylic acid is preferably at least one selected from the group consisting of fumaric acid, 2,2'-dithiodibenzoic acid, terephthalic acid, 4,4'-dihydroxybiphenyl-3,3'-dicarboxylic acid, and 2,5-furandicarboxylic acid.

Examples of the tricarboxylic acid include aconitic acid, trimellitic acid, trimesic acid (benzene tricarboxylic acid), biphenyl-3,4',5-tricarboxylic acid, and 1,3,5-tris(4-carboxyphenyl)benzene, and benzene tricarboxylic acid is particularly preferable.

The R is preferably any one of the following (A-1) to (A-11).

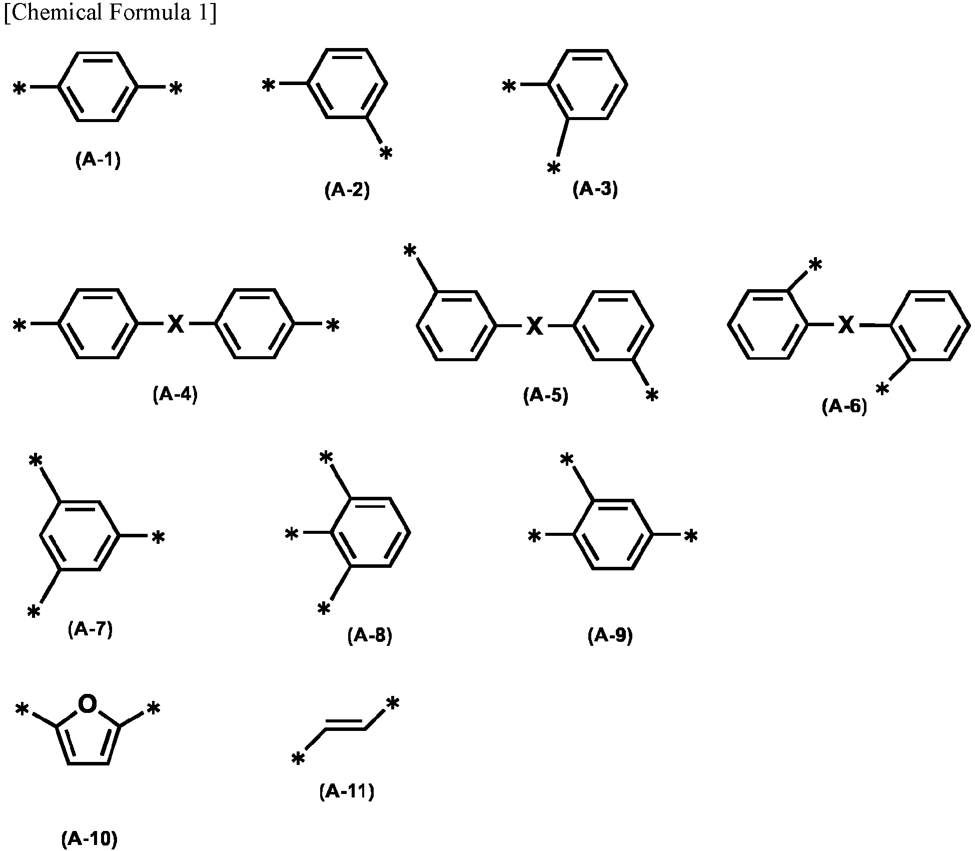

In the (A-1) to (A-11), at least one hydrogen atom bonded to a carbon atom may be substituted by -OH, and R bonds with (COO⁻) at *. In the formulas (A-4) to (A-6), -X- represents - S-S- or a single bond.

As the organic ligand constituting the MOF of the present invention, a different kind of an organic ligand other than carboxylates represented by the R(COO⁻)ₙ may be further included, and examples of the organic ligand include at least one selected from the group consisting of urea, pyrazine, oxazole, isoxazole, thiazole, imidazole, pyrazole, 1,2,3-thiadiazole, pyridazine, pyrimidine, purine, pteridine, 2,2'-bipyridine, and 4,4'-bipyridine, and 4,4'-bipyridine is particularly preferable.

The molar ratio (metal ion/organic ligand) of the metal ion to the organic ligand (in a case of a plurality of kinds of organic ligands, the total amount thereof) is preferably 0.1 or more, more preferably 0.5 or more, and further preferably 0.9 or more, and is preferably 10 or less, more preferably 5 or less, and further preferably 4 or less. The molar ratio is particularly preferably 0.9 or more and 4 or less.

Next, a method for producing the MOF of the present invention will be described. The MOF of the present invention can be obtained by reacting, in a solvent, a metal compound containing the metal ion constituting the MOF and one or more kinds of organic compounds for the organic ligands constituting the MOF. In addition, an inorganic compound for an inorganic ligand is also preferably mixed with the solvent together with the metal compound or the organic compounds, as necessary.

Specifically, it is preferable that a solution A obtained by completely dissolving, in a solvent, one of the metal compound containing the metal ion and the organic compounds for the organic ligands is prepared first and the other thereof is added dropwise (at this time, it is also preferable that a solution B obtained by dissolving the other thereof in a solvent is added dropwise). In addition, it is also preferable that a solution X obtained by completely dissolving, in a solvent, both the metal compound containing the metal ion and the organic compounds for the organic ligands is prepared, and then a different kind of a metal compound or a different kind of an organic compound other than the metal compound or the organic compounds in the solution X is added dropwise, or a solution Y obtained by completely dissolving, in a solvent, a different kind of a metal compound or a different kind of an organic compound other than the metal compound or the organic compounds in the solution X is added dropwise. The temperature during dropwise addition is preferably ordinary temperature (specifically, about 20 to 30°C). In addition, an amount of the metal compound or the organic compounds to be added dropwise is preferably 1.0 mmol/min. or less. The dropping rate is preferably 0.8 mmol/min. or less, more preferably 0.3mmol/min. or less, and may be 0.01 mmol/min. or more, , and may be 0.03 mmol/min. or more. In particular, the metal compound, or a solution obtained by dissolving the metal compound in a solvent is preferably added dropwise to a solution obtained by dissolving the organic compounds in a solvent, and, at this time, adding dropwise with the above-described dropping rate is further preferable.

The metal compound that contains the metal ion constituting the MOF is preferably metal sulfate, metal nitrate, metal acetate, metal chloride, metal bromide, or metal alkoxide, and metal nitrate, metal chloride, or metal bromide is particularly preferable.

The organic compounds for the organic ligands constituting the MOF include one or more selected from the group consisting of polyvalent carboxylic acids represented by R(COOH)ₙ. R and n are equivalent to R and n described above for the carboxylates represented by R(COO⁻)ₙ, and all the above descriptions about R and n, also including preferable aspects thereof, can be referred to.

The organic compounds for the organic ligands preferably include dicarboxylic acid or tricarboxylic acid. For specific examples of the dicarboxylic acid or the tricarboxylic acid, the dicarboxylic acid or the tricarboxylic acid described for the carboxylates represented by R(COO⁻)ₙ and preferable ranges thereof can be referred to.

As the organic compounds for the organic ligands, in addition to one or more selected from the group consisting of the polyvalent carboxylic acids represented by R(COOH)ₙ, at least one selected from the group consisting of urea, pyrazine, oxazole, isoxazole, thiazole, imidazole, pyrazole, 1,2,3-thiadiazole, pyridazine, pyrimidine, purine, pteridine, 2,2'-bipyridine, and 4,4'-bipyridine is preferably used. In addition, as the above-described inorganic compound for the inorganic ligand, alkali metal hydroxide or alkali metal azide is preferably used. Furthermore, in some cases, an inorganic ligand such as OH⁻, O²⁻, or OH₂ derived from the solvent for dissolving the metal compound and the organic compounds, water in the air, etc., is included in the MOF.

The solvent for dissolving the metal compound containing the metal ion or the organic compounds for the organic ligands is preferably water, an alcohol-based solvent such as methanol or ethanol, aliphatic acid-based solvent such as formic acid or acetic acid, or an amide-based solvent such as dimethylformamide, and only one of these solvents may be used or two or more of these solvents may be mixed and used.

In a case where the solution A, the solution B, or the solution X obtained by completely dissolving the metal compound containing the metal ion is prepared, each ratio of the metal compound to the solvent (metal compound/solvent) in the solution is preferably 0.01 mol/L or more, more preferably 0.1 mol/L or more, and is preferably 1 mol/L or less, more preferably 0.7 mol/L or less, further preferably 0.6 mol/L or less.

In a case where the solution A, the solution B, or the solution X obtained by completely dissolving the organic compounds for the organic ligands is prepared, each ratio of the organic compounds to the solvent (organic compounds/solvent) in the solution is preferably 0.01 mol/L or more, more preferably 0.02 mol/L or more, and further preferably 0.04 mol/L or more, and is preferably 1 mol/L or less, more preferably 0.7 mol/L or less, and further preferably 0.5 mol/L or less.

Furthermore, in a case where the inorganic compound for the inorganic ligand is contained in the solution A, the solution B, or the solution X, each ratio of the inorganic compound to the solvent (inorganic compound/solvent) in the solution is preferably 0.05 mol/L or more and is preferably 1 mol/L or less.

The molar ratio between the metal compound containing the metal ion and the organic compounds for the organic ligands may be adjusted such that the ratio of the molar quantity of metal atoms in the metal compound to the molar quantity of the organic compounds is equal to the above-described molar ratio of the metal ion to the organic ligands.

It is important that, after the above-described dropwise addition has finished, the resulting product is at least refluxed, stirred, or left as it is at a temperature of room temperature (e.g., 25°C) to 200°C for about 5 minutes to 100 hours. More specifically, it is important to react the metal compound and the organic compounds for 72 hours or more in a case where the temperature is room temperature to lower than 100°C, for 20 hours or more in a case where the temperature is 100°C or higher and lower than 130°C, and for 15 hours or more in a case where the temperature is 130°C or higher. The obtained reaction product is separated from the solvent through centrifugation, filtration, or the like, washed, and dried, whereby a desired MOF can be obtained.

This application claims priority to Japanese Patent Application No. 2022-174075 filed on October 31, 2022 and Japanese Patent Application No. 2022-174076 filed on October 31, 2022, the entire contents of which are incorporated herein by reference.

### EXAMPLES

The present invention will be described in more detail below by means of Examples. The present invention is not limited by the following Examples, and can also be carried out with appropriate modifications being made within the scope of the gist described above and below, and any of these modifications are included in the technical scope of the present invention.

### Example 1

In a 100 mL eggplant flask, 5.00 mmol of fumaric acid, 10 mL of DMF, and 3.5 mL of formic acid were mixed at 25°C and fumaric acid was completely dissolved, to obtain a solution A. Separately, 4.97 mmol of zirconyl chloride octahydrate, 10 mL of DMF, and 3.5 mL of formic acid were mixed at 25°C and zirconyl chloride octahydrate was completely dissolved to prepare a solution B, and the solution B was added dropwise to the solution A at 25°C over 1 hour. The ratio of fumaric acid to the solvent was 0.370 mol/L in the solution A, the ratio of zirconyl chloride octahydrate to the solvent was 0.368 mol/L in the solution B, and the dropping rate for zirconyl chloride octahydrate was 0.0828 mmol/min. Then, the resulting solution was left as it was at 130°C for 1 day, to obtain a suspension. The obtained precipitated solid product was subjected to three times of washing with 20 mL of DMF and filtering, and then was subjected to three times of washing with 20 mL of isopropanol and filtering. The obtained filter cake was dried under reduced pressure in a vacuum drying oven at 120°C for 24 hours, to obtain 1.00 g of a substance (yield: 84.2%).

### Example 2

In a 1 L eggplant flask, 3.05 mmol of cobalt(II) chloride hexahydrate, 10.42 mmol of 2,2'-dithiodibenzoic acid, 10.40 mmol of sodium azide, and 150 mL of DMF were mixed at 25°C, and cobalt(II) chloride hexahydrate, 2,2'-dithiodibenzoic acid and sodium azide were completely dissolved, to obtain a solution A. Separately, 3.08 mmol of benzene tricarboxylic acid, 3.10 mmol of 4,4'-bipyridine, and 150 ml of ethanol were mixed, and benzene tricarboxylic acid and 4,4'-bipyridine were completely dissolved, to prepare a solution B, and the solution B was added dropwise to the solution A at 25°C over 3 hours. The ratio of cobalt(II) chloride hexahydrate to the solvent was 0.0203 mol/L in the solution A, the ratio of 2,2'-dithiodibenzoic acid to the solvent was 0.0695 mol/L in the solution A, and the ratio of the total of the benzene tricarboxylic acid and 4,4'-bipyridine to the solvent was 0.0412 mol/L in the solution B. In addition, the dropping rate for the total amount of benzene tricarboxylic acid and 4,4'-bipyridine was 0.0343 mmol/min. Then, the resulting solution was stirred at 25°C for four days, to obtain a suspension. The obtained precipitated solid product was subjected to three times of washing with 30 mL of DMF and filtering, and the obtained filter cake was dried under reduced pressure in a vacuum drying oven at 120°C for 24 hours, to obtain 1.16 g of a substance (yield: 90.0%).

### Example 3

In a 100 mL pressure-resistant container (including a Teflon (registered trademark) inner cylinder) made of SUS-304, 0.50 mmol of terephthalic acid and 20 mL of methanol were mixed and terephthalic acid was completely dissolved, to obtain a solution A. A solution B obtained by mixing and completely dissolving 3.69 mmol of Al(NO₃)₃·9H₂O and 20 mL of methanol was added dropwise to the solution A at 25°C over 30 minutes. 1.57 g of 2 M NaOH in MeOH (0.2 g/2.5 ml) was added to the resulting solution, and the mixture was stirred by placing a stirrer chip for 5 minutes. The ratio of terephthalic acid to the solvent was 0.0250 mol/L in the solution A, the ratio of Al(NO₃)₃·9H₂O to the solvent was 0.185 mol/L in the solution B, and the dropping rate for Al(NO₃)₃·9H₂O was 0.123 mmol/min. Then, the resulting product was left as it was at 125°C for 20 hours. The obtained precipitated solid product was subjected to three times of washing with 30 mL of methanol and filtering, and the obtained filter cake was dried under reduced pressure in a vacuum drying oven at 120°C for 24 hours and at 150°C for 24 hours, to obtain 0.12 g of a substance (yield: 70.0%).

### Example 4

In a 50 mL eggplant flask, 5.04 mmol of 2,5-furandicarboxylic acid, 10.59 mmol of NaOH, and 15 mL of ion-exchanged water were mixed, and 2,5-furandicarboxylic acid and NaOH were completely dissolved, to obtain a solution A. A solution B obtained by mixing and completely dissolving 5.04 mmol of AlCl₃·6H₂O and 10 mL of ion-exchanged water was added dropwise to the solution A at 25°C over 30 minutes. The ratio of 2,5-furandicarboxylic acid to the solvent was 0.336 mol/L in the solution A, the ratio of AlCl₃·6H₂O to the solvent was 0.504 mol/L in the solution B, and the dropping rate for AlCl₃·6H₂O was 0.168 mmol/min. Then, the resulting solution was refluxed at 100°C for 24 hours, to obtain a suspension. The obtained precipitated solid product was subjected to three times of centrifugal washing with 50 mL of water, and the obtained cake was dried in a vacuum drying oven at 80°C for 24 hours, to obtain 0.67g of a substance (yield: 67%).

### Comparative Example 1

In a 50 mL pressure-resistant container (including a Teflon (registered trademark) inner cylinder) made of SUS-304, 3.0 mmol of 2-aminoterephthalic acid was completely dissolved in 25 mL of a mixture obtained by mixing dimethylformamide (DMF) and MeOH at a ratio of 1/1 (volume ratio), to obtain a solution A. 1.518 mmol of Ti[OCH(CH₃)₂]₄ was added to the solution A all at once at 25°C, and the resulting product was stirred by placing a stirrer chip for 5 minutes. Then, the resulting product was left as it was at 150°C for 16 hours. The obtained precipitated solid product was subjected to three times of washing with 10 mL of DMF and filtering, and the obtained filter cake was dried under reduced pressure in a vacuum drying oven at 120°C for 24 hours and at 150°C for 24 hours, to obtain 0.62 g of a substance (yield: 89.4%).

### Comparative Example 2

In a 100 mL pressure-resistant container (including a Teflon (registered trademark) inner cylinder) made of SUS-304, 7.483 mmol of terephthalic acid was completely dissolved in 25 mL of a mixture obtained by mixing dimethylformamide (DMF) and MeOH at a ratio of 9/1 (volume ratio), to obtain a solution A. The solution A was added dropwise to 4.523 mmol of Ti[OCH(CH₃)₂]₄ all at once at 25°C and mixed, and the resulting product was stirred by placing a stirrer chip for 5 minutes. Then, the resulting product was left as it was at 150°C for 16 hours. The obtained precipitated solid product was subjected to three times of washing with 10 mL of DMF and filtering, and the obtained filter cake was dried under reduced pressure in a vacuum drying oven at 120°C for 24 hours and at 150°C for 24 hours, to obtain 0.35 g of a substance (yield: 24.6%).

### Comparative Example 3

In a 100 mL pressure-resistant container (including a Teflon (registered trademark) inner cylinder) made of SUS-304, 3.178 mmol of 1H-1,2,3-triazole and 1.521 mmol of 2,5-thiophenedicarboxylic acid were dissolved in 56.64 g of dimethylformamide (DMF), to obtain a solution A. To the solution A, 5.268 mmol of Zn(NO₃)₂·6H₂O was added all at once at 25°C and mixed, and the resulting product was stirred by placing a stirrer chip for 20 minutes. Then, 1.522 mmol of terephthalic acid was further mixed at 25°C, and the resulting product was stirred by placing a stirrer chip for 20 minutes and was stirred at 100°C for 48 hours, to obtain a suspension. The obtained precipitated solid product was subjected to three times of washing with 30 mL of DMF and filtering, and the obtained filter cake was dried under reduced pressure in a vacuum drying oven at 120°C for 24 hours, to obtain 0.65 g of a substance (yield: 66.2%).

### Comparative Example 4

The same operation as in Example 4 was performed except that, after the solution B was added dropwise to the solution A, the resulting solution was refluxed at 25°C for 18 hours, to obtain a substance.

### (1) Measurement of porosity

The porosity was obtained as follows. Under the following conditions, each of the substances produced in Examples and Comparative Examples was measured using XRD to obtain a cif, and the cif was loaded into software named Mercury, whereby the porosity was obtained by inputting, a void contact surface having a probe radius of 1.5 Å and an approx. grid spacing of 0.7 Å, to the obtained data.
Device: SmartLab manufactured by Rigaku Corporation
Ray source: Cu
Measurement range: 20=3 to 40°
Step size: 0.01°
Scan speed: 3°/min
Measurement temperature: room temperature (25°C)

### (2) Measurement of largest pore diameter L and smallest pore diameter S

Based on rendering in Mercury which was obtained in the above-described measurement of the porosity, a crystal structure including pore size and pore shape was obtained, and a largest pore diameter L and a smallest pore diameter S were determined according to the above-described procedures (a1) to (a3).

### (3) Measurement of decomposition start temperature

The obtained MOF was held at 25°C for 12 hours under an air atmosphere in which the relative humidity was adjusted to 50% to perform pre-treatment. Then, the temperature was increased to 500°C at a temperature increase rate of 5°C/min. while nitrogen was caused to flow. According to DTA measurement defined in General rules for thermal analysis of JIS K0129, a point at which decomposition behavior was observed in this section (25 to 500°C) was set as a decomposition start temperature.

### (4) Measurement of water adsorption amount at relative pressure (P/P₀) (P represents water vapor pressure and P₀ represents saturation water vapor pressure)=0.1

Pre-treatment condition:
(i) Each sample was heated from 100°C to a temperature that was 50°C lower than a decomposition start temperature of the MOF, and was dried under reduced pressure for 12 hours;
(ii) The sample tube was connected to a pre-treatment device (BELPREP VACII), the air inside the sample tube was discharged, and then, N₂ gas (having a purity of 99.999% or more) was introduced therein until the pressure reached atmospheric pressure;
(iii) Thereafter, the sample tube was removed from the pre-treatment device, and the weight was measured by using a precision balance (that displays four or more decimal places) three times to obtain an average value (W1). When the precision balance was used, an ionizer was used to eliminate the effect of static electricity;
(iv) About 50 mg of the sample to be measured was weighed, and the sample was directly placed in a spherical part in the lower part of a standard sample tube by using a long stem funnel;
(v) A glass bar was returned into the sample tube, the sample tube was sealed with a quick seal, and then the total weight was measured once and the amount of the added sample was tentatively checked. The sample tube containing the sample was connected to the pre-treatment device to exhaust gas from the sample tube; and
(vi) After the pressure in the sample tube became sufficiently low, heating was started (while vacuuming was continued).
Measurement device: BELSORP-max manufactured by MicrotracBEL

Measurement condition:
(i) A water adsorption amount as a weight percent (wt%) was calculated as (water adsorption amount/(amount of measurement sample)×100), by inputting the weight of the sample and information on water at a measurement temperature (saturation vapor pressure, etc.) to the measurement software, setting the measurement relative pressure, at which measurement was desired to be performed, etc., and starting the measurement;
(ii) Ultrapure water which was an adsorbate was placed into a solvent reservoir, and the solvent reservoir was connected to the device. Then, defoaming treatment (the solvent reservoir was immersed in liquid nitrogen filled in a Dewar vessel to freeze the ultrapure water;
(iii) After it was confirmed that the ultrapure water was sufficiently frozen, a valve in the upper part of the solvent reservoir was opened to evacuate the solvent reservoir. The valve in the upper part of the solvent reservoir was closed. The frozen water was melted, and the air contained in the ultrapure water was taken out as gas) was performed at least three times to complete preparation of the adsorbate;
(iv) The weight of the sample, and information on water at a measurement temperature (saturation vapor pressure, etc.) were inputted to the measurement software, the measurement relative pressure, at which measurement was desired to be performed, etc. were set, and a measurement start button was pressed;
(v) Thereafter, according to instructions of the software, the Dewar vessel filled with liquid nitrogen or the sample tube was set, and measurement was performed; and
(vi) Based on the obtained result, a water adsorption amount was derived at relative pressure (P/P0) (P represents water vapor pressure and P0 represents saturation water vapor pressure)=0.1.

The results are shown in Table 1.

**[Table 1]**

| | Metal ion | Organic ligand | Metal ion/ Organic ligand (molar ratio) | Largest pore diameter L (Å)" | Smallest pore diameter S (Å) | Largest pore diameter L / Smallest pore diameter S | Decomposition start temperature (°C) | Water adsorption amount at relative pressure (P/P₀)=0.1 (ml/g) |
|---|---|---|---|---|---|---|---|---|
| Example 1 | Zr | Fumaric acid | 0.99 | 7.31 | 5.66 | 1.29 | 250 | 150 |
| Example 2 | Co | Benzene tricarboxylic acid | 0.18 | 3.70 | 3.16 | 1.17 | 250 | 54 |
| | | 2,2'-dithiodibenzoic acid | | | | | | |
| | | 4,4'-bipyridine | | | | | | |
| Example 3 | Al | Terephthalic acid | 7.38 | 6.57 | 5.96 | 1.10 | 250 | 79 |
| Example 4 | Al | 2,5-furandicarboxylic acid | 1.00 | 3.76 | 3.18 | 1.18 | 220 | 53 |
| Comparative example 1 | Ti | 2-aminoterephthalic acid | 0.51 | 4.54 | 4.09 | 1.11 | 200 | 32 |
| Comparative example 2 | Ti | Terephthalic acid | 0.60 | 4.37 | 4.02 | 1.09 | 200 | 26 |
| Comparative example 3 | Zn | 2,5-thiophenedicarboxylic acid | 0.85 | 13.75 | 4.87 | 2.82 | 200 | 21 |
| | | Terephthalic acid | | | | | | |
| | | Triazole | | | | | | |
| Comparative example 4 | Al | 2,5-furandicarboxylic acid | 1.00 | Crystalline substance could not be confirmed. | | | | |

In each of Examples 1 to 4, a crystalline MOF having the largest pore diameter L/the smallest pore diameter S of 1.0 to 2.0 and a decomposition start temperature of higher than 200°C was obtained. However, in each of Comparative Examples 1 to 3, a MOF that did not satisfy at least one of requirements with respect to the largest pore diameter L/the smallest pore diameter and the decomposition start temperature was obtained. In Comparative Example 4, the reflux condition applied after the solution B was added dropwise to the solution A was not appropriate, so that a crystalline substance could not be confirmed in measurement using XRD. The through pore Pₘₐₓ observed in each of Example 2, Example 3, Comparative Example 1, Comparative Example 2, and Comparative Example 3 intersected with another through pore.

### INDUSTRIAL APPLICABILITY

The MOF of the present invention can be suitably used to adsorb and remove gas or organic molecules, for example. Examples of the gas include water (water vapor), carbon dioxide, hydrogen, carbon monoxide, oxygen, nitrogen, hydrocarbon having 1 to 4 carbon atoms, noble gas, hydrogen sulfide, ammonia, sulfur oxide, nitrogen oxide, and siloxane. Examples of the organic molecule include hydrocarbon having 5 to 8 carbon atoms, alcohol having 1 to 8 carbon atoms, aldehyde having 1 to 8 carbon atoms, carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. The organic molecule may contain an aromatic ring.

### DESCRIPTION OF THE REFERENCE CHARACTERS

11 inscribed circle
21 cross section
22 through pore Pₘₐₓ
23a, 23b outer perimeter of through pore Pₘₐₓ
24 central axis of through pore Pₘₐₓ
25 largest pore diameter L
26 smallest pore diameter S

## Claims

1. A metal organic framework comprising
an organic ligand and a metal ion, wherein
the organic ligand includes an organic ligand having a group represented by R(COO⁻)ₙ in which R represents at least one of an unsaturated straight-chain hydrocarbon group, an aromatic hydrocarbon group, and a hydrocarbon group that has an aromatic ring including an oxygen atom and may have a functional group X which is -OH or -S-S-, and n is 2 or more and 3 or less,
the metal ion is an ion of at least one metal selected from the group consisting of Al, Ga, In, Ti, V, Cr, Mn, Fe, Co, Cu, Zr, and Hf,
a ratio of a largest pore diameter L to a smallest pore diameter S (the largest pore diameter L/the smallest pore diameter S) determined through the following procedures (a1) to (a3) is 1.0 to 2.0, and
a decomposition start temperature is higher than 200°C,
(a1) a crystal structure determined through X-ray crystal structure analysis is displayed using a space-filling model, and is displayed so as to include outer perimeters of through pores without any omissions, and presence or absence of the through pores is observed from all directions, to identify a through pore Pₘₐₓ having the largest inscribed circle diameter;
(a2) in each of a cross section ab, a cross section bc, and a cross section ca of the through pore Pₘₐₓ, a longest length is found among inner diameters of the through pore Pₘₐₓ obtained by cutting the through pore Pₘₐₓ along lines orthogonal to a central axis of the through pore Pₘₐₓ, and a longest one, among the longest length in the cross section ab, the longest length in the cross section bc, and the longest length in the cross section ca, is set as the largest pore diameter L of the through pore Pₘₐₓ,
the cross section ab being a cut cross section, of the through pore Pₘₐₓ, along a plane parallel to an a-b plane of a unit cell, and being a plane obtained by cutting the through pore Pₘₐₓ where the through pore Pₘₐₓ penetrates,
if a plurality of the cross sections along which the through pore Pₘₐₓ can be cut where the through pore Pₘₐₓ penetrates are present, the cross section ab being the cross section in which an overlapping portion with the through pore Pₘₐₓ is the largest,
the cross section bc being a cross section obtained by replacing the cross section ab with the cross section bc and the a-b plane with a b-c plane, and the cross section ca being a cross section obtained by replacing the cross section ab with the cross section ca and the a-b plane with a c-a plane, in identifying of the cross section ab; and
(a3) in the cross section in which the largest pore diameter L is determined, a smallest length, among inner diameters of the through pore Pₘₐₓ obtained by cutting the through pore Pₘₐₓ along the lines orthogonal to the central axis of the through pore Pₘₐₓ, is set as the smallest pore diameter S.

2. The metal organic framework according to claim 1, wherein
a through pore observed when a crystal structure of a metal organic framework determined through X-ray crystal structure analysis is displayed using a space-filling model does not intersect with another through pore.
